Europäisches Patentamt

European Patent Office

Office européen des brevets

(19)

(11) **EP 0 634 136 B1**

(12) **EUROPEAN PATENT SPECIFICATION**

(45) Date of publication and mention
of the grant of the patent:
**18.11.1998 Bulletin 1998/47**

(51) Int Cl.⁶: **G06F 17/00**

(21) Application number: **94109315.5**

(22) Date of filing: **16.06.1994**

(54) **Method and device for processing ECG-signals**

Methode und Vorrichtung zum Verarbeiten von EKG-Signalen

Méthode et dispositif pour traiter des signaux d'ECG

(84) Designated Contracting States:
**AT BE CH DE DK ES FR GB IT LI NL SE**

(30) Priority: **16.07.1993 SE 9302435**

(43) Date of publication of application:
**18.01.1995 Bulletin 1995/03**

(73) Proprietor: **Siemens-Elema AB**
**171 95 Solna 1 (SE)**

(72) Inventors:
• **Ohlsson, Thomas**
  **S-165 77 Hässelby (SE)**
• **Atarius, Roozbeh**
  **S-224 68 Lund (SE)**
• **Sörnmo, Leif**
  **S-224 65 Lund (SE)**

(56) References cited:
**US-A- 4 732 158**          **US-A- 4 796 638**
**US-A- 5 020 540**          **US-A- 5 211 178**

• **American journal of physiology, Volume 244,
  April 1983, Steven L. Blumlein et al, "New
  technique for detection of changes in QRS
  morphology of ECG signals pp 560-566"**

## Description

The present invention relates to a method and a device for processing ECG-signals for the purpose of detecting low-amplitude signal structures in noise of the kind described in the preambles of claims 1 and 13 respectively.

These signal structures, such as so-called late potentials, have an amplitude of the order of a few µV, usually making impossible direct, quantitative detection of the same in the ECG-signal. Signal averaging has hitherto been frequently used to reduce the level of noise. The signal is further subjected to highpass filtration in order to eliminate low-frequency components. The vector quantities of the X, Y and Z leads is then compared to a threshold value so as to determine whether or not late potentials are present, cf. US,A,4 422 459. However, residual noise affects the vector quantity, thereby degrading accuracy. Important information in individual leads is also lost when they are combined into a single vector quantity.

Spectral analysis of late potentials has also appeared to be associated with major difficulties, since spectral analysis of predominantly transient signals is generally complicated.

The object of the present invention is to propose a new method and provide a new device, based on a statistical model which explicitly takes the presence of noise into account, for more reliable detection of low-amplitude signal structures in ECG-signals.

This object is achieved with a method and a device of the kind mentioned in the introductory part having the characterizing features set forth in claims 1 and 13 respectively.

The present invention utilizes the circumstance that noise is essentially uncorrelated from one cardiac cycle to another, whereas the ECG-activity of interest is repetitive.

According to one advantageous further development of the invention, at least two sub-average values are formed of at least the part of the signals containing the signal structure in question from at least two different groups of cardiac cycles in the plurality of cardiac cycles, whereupon the concordance between the sub-average values is determined. This improves the possibility of separating the signal from noise.

In the present invention each lead is processed individually, thereby avoiding the loss of information which occurs when a plurality of leads is combined into a single vector unit.

The invention will now be described in greater detail with the aid of an exemplifying embodiment of the device according to the invention with reference to the attached drawings in which

FIG. 1 is an overview block diagram of the device according to the invention;

FIG. 2 is a block diagram of the unit for identifying low-amplitude signals in the presence of noise, and

FIG. 3 illustrates, also in the form of a block diagram, in greater detail so-called eigenvector filtration and subsequent non-linear signal processing in order to provide a function suitable for determining the end time of late potentials.

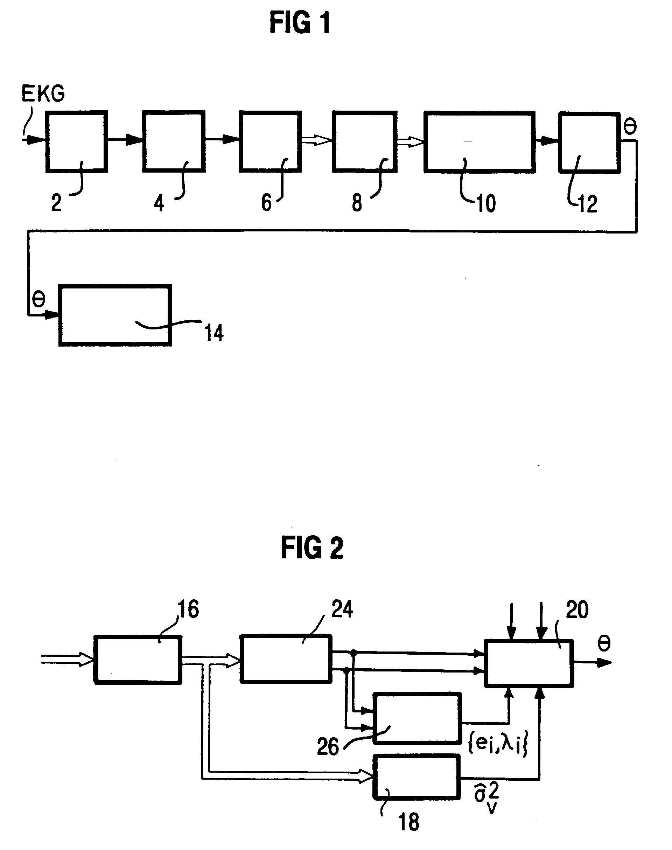

The recorded ECG-signals are converted into digital form in the A/D converter 2, and the QRS complexes are detected in the detector 4 in FIG. 1. In the averager 6 are formed at least two sub-average values of the signals, and in the unit 8 is set an interval or a time window which is utilized in the subsequent unit 10 for determining the possible presence of a signal in the noise environment. The unit 10 comprises a calculator unit for calculating the cross-covariance of the sub-average values received from the averager 6 in successive, overlapping time intervals set in the unit 8. These calculations result in a cross-covariance matrix which determines the eigenvectors utilized for eigenvector-based filtration of the sub-average values for the purpose of filtering signals out of the noise. This is described in greater detail below.

After the unit 10 follows an additional means 12 for non-linear processing of the accordingly filtered signals, so an output signal is achieved which essentially represents the signal amplitude. The end time of the signal is finally determined in the unit 14 from the chronological course of the signal amplitude curve obtained.

The unit 14 comprises a comparator unit which compares the terminal part of the signal amplitude curve with a predetermined standard curve which is successively displaced in relation to the signal amplitude curve so the time at which the concordance between the standard curve and the signal amplitude curve is best can be determined, which time is set as the end time for the late potentials.

In FIG. 2 the construction of the units 6, 8, 10, 12 in FIG. 1 is described in greater detail.

After time-synchronization of the signals from individual cardiac cycles, the signals are processed in the signal processing unit 16, e.g. high-pass filtered to remove low-frequency components.

In the unit 18, an estimate is made of the noise a in the signals from the unit 16, and this noise estimate is supplied to the unit 20.

The processed signals are also supplied to an averager 24, to form at least two sub-average values, as above, and then to a calculator unit 26 for calculation of the cross-covariance between the sub-average values. The cross-covariance calculations result in a cross-covariance matrix the eigenvectors $e_i$ and eigenvalue $\lambda_i$ of which as well as the sub-average values obtained from the averager 24, are supplied to the unit 20.

The signals are supplied to the averager 24 in the form of an ensemble of time-synchronized signals from a plurality of cardiac cycles. One sub-average value is formed by calculation in successive, overlapping intervals or time windows of every other signal in the ensemble, and the other sub-average value is formed by corresponding calculation of the intermediate signals in the ensemble. Every time window is long enough to cover at least two samples.

However, this is only one example of the way in which averaging can be performed. It can obviously be carried out in countless different ways. Thus, too differing signals in the signal ensemble could e.g. be excluded in determination of average values, and the determination of average values need not be performed in successive, overlapping intervals, as mentioned in the example above. Averaging does not have to be performed using every other signal of the ensemble, but the signals in the ensemble could be divided into two groups in some other way, whereupon average values could be formed of the signals in the groups.

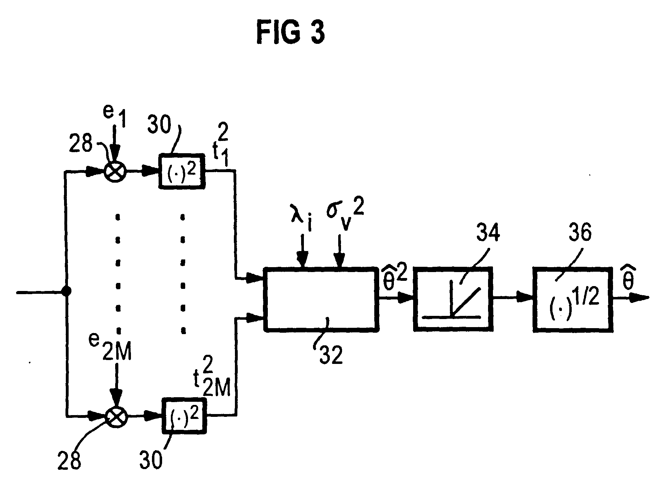

The unit 20 in FIG. 2 is shown in greater detail in FIG. 3.

At 28 in FIG. 3, the eigenvectors of the cross-covariance matrix $e_1$, $e_2$ ... $e_{2M}$ are used for eigenvector-based filtration of the sub-average values. Filtering the sub-average values with the most significant eigenvectors, or even only the most significant eigenvector, i.e. the vector or vectors corresponding to the largest eigenvalues, is often sufficient. In practice, therefore, filtration with a limited number of eigenvectors is enough.

After the filtration the signals are supplied to the squarer 30 which delivers output signals corresponding to the quantities $t_1^2$, $t_2^2$ ... $t_{2M}^2$ where $t_i$ denotes the correlation between the subaverage values and the i:th eigenvector.

The outputs of the squarer 30 are connected to a so-called Maximum Likelihood estimator 32 to which the noise variance $\sigma^2$, and the eigenvalues from the cross-covariance matrix are fed from the unit 18 for noise estimation and from the unit 26 for calculating the cross-covariance in FIG. 2 respectively.

The signal effect $\Theta^2$ is determined in the estimator 32 by solving the equation

$$\sum_{i=1}^{2m} \lambda_i \left( \frac{1}{\Theta^2 \lambda_i + \sigma_v^2} - \frac{t_i^2}{\left(\Theta^2 \lambda_i + \sigma_v^2\right)^2} \right) = 0$$

in which

$\lambda_i$      designates eigenvalues of the cross-covariance matrix,
$\sigma_v^2$      the variance of the estimated noise,
$t_i$      the correlation between the sub-average values and the i-th column in the eigenvector matrix
$\Theta^2$      the signal effect, and
$m$      the number of samples in the interval.

Since $\Theta^2$ is not always greater than zeros, $\Theta^2$ is supplied to a unit 34 with diode-like characteristic which gives an output signal which is equal to zero for negative $\Theta^2$-values and an output signal directly proportional to $\Theta^2$ for positive $\Theta^2$-values, whereupon the square root of this quantity is calculated in the unit 36. The output signal for the unit 36 represents the signal amplitude $\Theta$.

Thus, the present invention makes possible very effective detection of low-amplitude ECG-activity in a noise environment, correlation in both time and over an ensemble of cardiac cycles being taken into account.

## Claims

1. Method for processing ECG-signals for the purpose of detecting low-amplitude signal structures in noise, in which method ECG-signals from a plurality of cardiac cycles are A/D converted, and QRS complexes are detected, characterized in that the signals from at least two cardiac cycles are timesynchronized, whereupon the concordance between these signals in time intervals encompassing at least two samples is determined, and an output quantity representing the concordance thus determined is generated, the magnitude of the output quantity designating the amplitude of the signal structures in question.

2. Method according to claim 1, **characterized** in that the noise level is estimated of the signals from each heart cycle for compensation at the determination of the concordance.

EP 0 634 136 B1

3. Method according to any of claims 1 or 2, **characterized** in that at least two sub-average values are formed of at least the part of the signals containing the signal structure in question from at least two different groups of cardiac cycles in said plurality of cardiac cycles, whereupon the concordance between the sub-average values is determined.

4. Method according to claim 3, **characterized in** that n sub-average values, where n>2, are determined from the signals of the cardiac cycles 1, n+1, 2n+1 ... and 2, n+2, 2n+2 etc. and n; 2n, 3n ... respectively of the said plurality of cardiac cycles.

5. Method according to any of claims 3 or 4, **characterized** in that the cross-covariance of the sub-average values is determined in order to determine the concordance between these values.

6. Method according to claim 5, **characterized** in that the cross-covariance is calculated for the sub-average values in successive, overlapping intervals.

7. Method according to any of claims 5 or 6, **characterized** in that the sub-average values are filtered according to the magnitude of cross-covariance, whereupon the filtered values are subjected to non-linear processing to form a function representing the amplitude of said signal structures.

8. Method according to claim 7, **characterized** in that the noise compensation of the filtered sub-average values is performed in conjunction with the non-linear signal processing.

9. Method according to any of claims 5 - 8, **characterized** in that the concordance of the sub-average values is represented by a cross-covariance matrix resulting from the calculation of cross-covariance, and in that the filtration of the sub-average values is performed as an eigenvector-based filtration with eigenvectors determined from the cross-covariance matrix.

10. Method according to any of claims 7 - 9, **characterized** in that the non-linear processing comprises squaring of the filtered sub-average values, calculation of the signal power and calculation therefrom of the signal amplitude.

11. Method according to claim 10, **characterized** in that the resulting signal amplitude curve is compared with a standard curve for determining a characteristic time in the signal structure in question, e.g. its end point.

12. Method according to any of claims 1 - 11, **characterized** in that the said signal structures comprise so-called late potentials.

13. Device for processing ECG-signals for the purpose of detecting low-amplitude signal structures in noise, comprising means (2,4) for recording ECG-signals from a plurality of cardiac cycles, A/D-conversion of the signals and detection of QRS complexes, **characterized** in that a synchronization unit is arranged to time-synchronize the signals from at least two cardiac cycles, and in that a calculator unit (10) is arranged to determine the concordance between the signals in time intervals, comprising at least two samples, and in that output means (12) are connected to the calculator unit in order to generate an output quantity the magnitude of which designates the amplitude of the signal structures in question.

14. Device according to claim 13, **characterized** in that an averager (6) is arranged to form at least two sub-average values of at least the parts of the ECG-signals, which comprise said signal structures, in at least two different groups of cardiac cycles of said plurality of heart cycles, said averager (6) being connected to the calculator unit (10) for determining the concordance between the sub-average values.

15. Device according to claim 14, **characterized** in that the calculator unit (10) is arranged to form the cross--covariance between the sub-average values in order to determine the concordance.

16. Device according to claim 15, **characterized** in that the calculator unit (10) is connected to a filter unit (16) for filtering the sub-average values according to the magnitude of cross-covariance, and in that a signal processing unit (12) is arranged to subject the filtered values to a non-linear processing in order to form a function representing the amplitude of said signal structures.

17. Device according to claim 16, **characterized** in that a unit (18) is arranged to estimate the level of noise in the

4

signal from each cardiac cycle, which unit is connected to the signal processing unit (10) in order to compensate the sub-average values for the noise in conjunction with the non-linear signal processing.

18. Device according to any of claims 15 - 17, **characterized** in that the calculator unit (10) is arranged to calculate the cross-covariance of the sub-average values in successive, overlapping intervals.

19. Device according to any of claims 16 - 18, **characterized** in that the calculator unit (10) controls the filter unit (16) to perform eigenvector-based filtration, controlled by eigenvectors determined by the cross-covariance calculation.

20. Device according to any of claims 16 - 19, **characterized** in that the signal processing unit (12) is arranged to square the filtered sub-average values and determine the signal power and therefrom the signal amplitude.

21. Device according to patent claim 20, **characterized** in that the signal processing unit (12) comprises a so-called Maximum Likelihood estimator (32), arranged to solve the Maximum Likelihood equation

$$\sum_{i=1}^{2m} \lambda_i \left( \frac{1}{\Theta^2 \lambda_i + \sigma_v^2} - \frac{t_i^2}{\left( \Theta^2 \lambda_i + \sigma_v^2 \right)^2} \right) = 0$$

in which

$\lambda_i$ denotes eigenvalues of the cross-covariance matrix,
$\sigma_v^2$ the variance of the estimated noise,
$t_i$ the correlation between sub-average values and i:th column in the eigenvector matrix,
$\Theta^2$ the signal power, and
m the number of samples in the interval.

22. Device according to any of the claims 16 - 21, **characterized** in that a comparator unit (14) is connected to the output of the signal processing unit (12) in order to compare the terminal part of the signal amplitude curve with a predetermined standard curve in order to determine a characteristic time in the signal structure as the time position in the standard curve which gives the best concordance with the signal amplitude curve.

**Patentansprüche**

1. Methode zur Verarbeitung von EKG-Signalen zum Zweck, Signalstrukturen mit niedriger Amplitude im Rauschen zu detektieren, bei der EKG-Signale von einer Vielzahl von Herzzyklen Analog/Digital umgewandelt werden, **dadurch gekennzeichnet,** daß die Signale von mindestens zwei Herzzyklen zeitsynchronisiert werden, woraufhin die Konkordanz zwischen diesen Signalen in Zeitintervallen bestimmt wird, die mindestens zwei Proben umfassen, und eine Ausgangsquantität erzeugt wird, die die auf diese Weise bestimmte Konkordanz repräsentiert und die Größe der Ausgangsquantität die Amplitude der betreffenden Signalstrukturen bestimmt.

2. Methode nach Anspruch 1, **dadurch gekennzeichnet,** daß das Rauschniveau der Signale von jedem Herzzyklus zum Zwecke der Kompensation bei der Bestimmung der Konkordanz abgeschätzt wird.

3. Methode nach einem der Ansprüche 1 oder 2, **dadurch gekennzeichnet,** daß zumindest zwei Untermittelwerte aus zumindest dem die betreffende Signalstruktur enthaltenden Teil der Signale von mindestens zwei verschiedenen Gruppen von Herzzyklen aus der Vielzahl von Herzzyklen gebildet werden, woraufhin die Konkordanz zwischen den Untermittelwerten bestimmt wird.

4. Methode nach Anspruch 3, **dadurch gekennzeichnet**, daß n Untermittelwerte, wobei n > 2 ist, aus den Signalen der Herzzyklen 1, n+1, 2n+1 ... und 2, n+2, 2n+2 usw. beziehungsweise n, 2n, 3n ... der Vielzahl von Herzzyklen bestimmt werden.

5. Methode nach einem der Ansprüche 3 oder 4, **dadurch gekennzeichnet**, daß die Kreuzkovarianz der Untermittelwerte bestimmt wird, um die Konkordanz zwischen diesen Werten zu bestimmen.

6. Methode nach Anspruch 5, **dadurch gekennzeichnet,** daß die Kreuzkovarianz für die Untermittelwerte in aufeinanderfolgenden, überlappenden Intervallen berechnet wird.

7. Methode nach einem der Ansprüche 5 oder 6, **dadurch gekennzeichnet,** daß die Untermittelwerte entsprechend der Größe der Kreuzkovarianz gefiltert werden, woraufhin die gefilterten Werte einer nichtlinearen Verarbeitung unterzogen werden, um eine Funktion zu bilden, die die Amplitude der Signalstrukturen repräsentiert.

8. Methode nach Anspruch 7, **dadurch gekennzeichnet,** daß die Rauschkompensation der gefilterten Untermittelwerte in Verbindung mit der nichtlinearen Signalverarbeitung durchgeführt wird.

9. Methode nach einem der Ansprüche 5 - 8, **dadurch gekennzeichnet,** daß die Konkordanz der Untermittelwerte durch eine aus der Berechnung der Kreuzkovarianz resultierenden Kreuzkovarianzmatrix repräsentiert wird und daß das Filtern der Untermittelwerte als ein eigenvektorbasiertes Filtern mit aus der Kreuzkovarianzmatrix bestimmten Eigenvektoren durchgeführt wird.

10. Methode nach einem der Ansprüche 7 - 9, **dadurch gekennzeichnet**, daß die nichtlineare Verarbeitung das Quadrieren der gefilterten Untermittelwerte, die Berechnung der Signalstärke und die Berechnung der Signalamplitude daraus umfaßt.

11. Methode nach Anspruch 10, **dadurch gekennzeichnet,** daß die resultierende Signalamplitudenkurve mit einer Standardkurve zum Bestimmen einer charakteristischen Zeit in der betreffenden Signalstruktur, z.B. ihres Endpunktes, verglichen wird.

12. Methode nach einem der Ansprüche 1 - 11, **dadurch gekennzeichnet,** daß die Signalstruktur sogenannte Spätpotentiale enthält.

13. Vorrichtung zur Verarbeitung von EKG-Signalen zum Zweck, Signalstrukturen mit niedriger Amplitude in Rauschen zu detektieren, mit Mitteln (2,4) zum Aufzeichnen von EKG-Signalen aus einer Vielzahl von Herzzyklen, Analog/Digital-Umwandlung der Signale und Detektion von QRS-Komplexen, **dadurch gekennzeichnet,** daß eine Synchronisationseinheit angeordnet ist, um die Signale von mindestens zwei Herzzyklen zeitlich zu sychronisieren, und daß eine Berechnungseinheit (10) zur Bestimmung der Konkordanz zwischen den Signalen in Zeitintervallen, die mindestens zwei Proben enthalten, vorgesehen ist, und daß Ausgangsmittel (12) mit der Berechnungseinheit verbunden sind, um eine Ausgangsquantität zu erzeugen, deren Größe die Amplitude der betreffenden Signalstruktur bestimmt.

14. Vorrichtung nach Anspruch 13, **dadurch gekennzeichnet,** daß ein Mittelwertbildner (6) vorgesehen ist, um mindestens zwei Untermittelwerte von mindestens Teilen der EKG-Signale, die die Signalstrukturen enthalten, in mindestens zwei verschiedenen Gruppen von Herzzyklen der Vielzahl von Herzzyklen zu bilden, wobei der Mittelwertbildner (6) mit der Berechnungseinheit (10) zur Bestimmung der Konkordanz zwischen den Untermittelwerten verbunden ist.

15. Vorrichtung nach Anspruch 14, **dadurch gekennzeichnet,** daß die Berechnungseinheit (10) dazu ausgebildet ist, die Kreuzkovarinaz zwischen den Untermittelwerten zu bilden, um die Konkordanz zu bestimmen.

16. Vorrichtung nach Anspruch 15, **dadurch gekennzeichnet,** daß die Berechnungseinheit (10) mit einer Filtereinheit (16) zum Filtern der Untermittelwerte entsprechend der Größe der Kreuzkovarianz verbunden ist und daß eine Signalverarbeitungseinheit (12) vorgesehen ist, um die gefilterten Werte einer nichtlinearen Verarbeitung zu unterziehen, um eine die Amplitude der Signalstrukturen repräsentierende Funktion zu bilden.

17. Vorrichtung nach Anspruch 16, **dadurch gekennzeichnet,** daß eine Einheit (18) zum Abschätzen des Rauschniveaus in dem Signal von jedem Herzzyklus vorgesehen ist, die mit der Signalverarbeitungseinheit (10) verbunden ist, um dieses Rauschen in den Untermittelwerte in Verbindung mit der nichtlinearen Signalverarbeitung zu kompensieren.

18. Vorrichtung nach einem der Ansprüche 15 - 17, **dadurch gekennzeichnet**, daß die Berechnungseinheit (10) zum Berechnen der Kreuzkovarinaz der Untermittelwerte in aufeinanderfolgenden, überlappenden Intervallen vorgesehen ist.

**19.** Vorrichtung nach einem der Ansprüche 16 - 18, **dadurch gekennzeichnet**, daß die Berechnungseinheit (10) die Filtereinheit (16) steuert, um eine eigenvektorbasierte Filterung, die durch die durch die Kreukovarinazberechnung bestimmten Eigenvektoren gesteuert wird, durchzuführen.

**20.** Vorrichtung nach einem der Ansprüche 16 - 19, **dadurch gekennzeichnet**, daß die Signalverarbeitungseinheit (12) zum Quadrieren der gefilterten Untermittelwerte und zum Bestimmen der Signalstärke und daraus der Signalamplitude vorgesehen ist.

**21.** Vorrichtung nach Anspruch 20, **dadurch gekennzeichnet,** daß die Signalverarbeitungseinheit (12) einen sogenannten Kalkulator größter Wahrscheinlichkeit (32) aufweist, der dazu vorgesehen ist, die Gleichung größter Wahrscheinlichkeit

$$\sum_{i=1}^{2m} \lambda_i \left( \frac{1}{\Theta^2 \lambda_i + \sigma_v^2} - \frac{t_i^2}{\left( \Theta^2 \lambda_i + \sigma_v^2 \right)^2} \right) = 0$$

zu lösen, in der

$\lambda_i$ Eigenwerte der Kreuzkovarianzmatrix,
$\sigma_v^2$ die Varianz des abgeschätzten Rauschens,
$t_i$ die Korrelation zwischen Untermittelwerten und der i-ten Spalte in der Eigenvektormatrix,
$\Theta^2$ die Signalstärke, und
m die Anzahl der Proben in dem Intervall bezeichnen.

**22.** Vorrichtung nach einem der Ansprüche 16 - 21, **dadurch gekennzeichnet**, daß die Komparatoreinheit (14) mit dem Ausgang der Signalverarbeitungseinheit (12) verbunden ist, um den Endteil der Signalamplitudenkurve mit einer vorbestimmten Standardkurve zu vergleichen, um eine charakteristische Zeit in der Signalstruktur als die Zeitposition in der Standardkurve, die die beste Konkordanz mit der Signalamplitudenkurve ergibt, zu bestimmen.

**Revendications**

**1.** Procédé pour traiter des signaux d'ECG dans le but de détecter des structures de signaux à faible amplitude dans du bruit, dans lequel procédé les signaux d'ECG provenant d'une pluralité de cycles cardiaques sont convertis d'analogique en numérique et les complexes QRS sont détectés, caractérisé en ce que les signaux provenant d'au moins deux cycles cardiaques sont synchronisés temporellement, sur quoi la concordance entre ces signaux dans les intervalles de temps enfermant au moins deux échantillons est déterminée et une quantité de sortie représentant la concordance ainsi déterminée est générée, l'amplitude de la quantité de sortie désignant l'amplitude des structures des signaux en question.

**2.** Procédé selon la revendication 1, caractérisé en ce que le niveau du bruit est l'estimée des signaux provenant de chaque cycle cardiaque pour une compensation au moment de la détermination de la concordance.

**3.** Procédé selon l'une quelconque des revendications 1 ou 2, caractérisé en ce qu'au moins deux valeurs de sous-moyenne sont formées d'au moins la partie des signaux contenant la structure des signaux en question issue d'au moins deux groupes différents des cycles cardiaques dans ladite pluralité des cycles cardiaques, sur quoi la concordance entre les valeurs de sous-moyenne est déterminée.

**4.** Procédé selon la revendication 3, caractérisé en ce que les n valeurs de sous-moyenne, où n>2, sont déterminées à partir des signaux des cycles cardiaques 1, n + 1, 2n + 1 ... et 2, n+2, 2n + 2, etc. et n ; 2n, 3n ... respectivement de ladite pluralité de cycles cardiaques.

5. Procédé selon l'une quelconque des revendications 3 ou 4, caractérisé en ce que la covariance croisée des valeurs de sous-moyenne est déterminée afin de déterminer la concordance entre ces valeurs.

6. Procédé selon la revendication 5, caractérisé en ce que la covariance croisée est calculée pour les valeurs de sous-moyenne dans des intervalles successifs se chevauchant.

7. Procédé selon l'une quelconque des revendications 5 ou 6, caractérisé en ce que les valeurs de sous-moyenne sont filtrées en conformité avec l'amplitude de la covariance croisée, sur quoi les valeurs filtrées sont soumises à un traitement non-linéaire afin de former une fonction représentant l'amplitude desdites structures des signaux.

8. Procédé selon la revendication 7, caractérisé en ce que la compensation du bruit des valeurs de sous-moyenne filtrées est effectuée en liaison avec le traitement des signaux non-linéaire.

9. Procédé selon l'une quelconque des revendications 5 à 8, caractérisé en ce que la concordance des valeurs de sous-moyenne est représentée par une matrice de covariances croisées résultant du calcul de la covariance croisée et en ce que le filtrage des valeurs de sous-moyenne est effectué comme un filtrage à base de vecteurs propres, les vecteurs propres étant déterminés à partir de la matrice de covariances croisées.

10. Procédé selon l'une quelconque des revendications 7 à 9, caractérisé en ce que le traitement non-linéaire comprend l'élévation au carré des valeurs de sous-moyenne filtrées, le calcul de la puissance des signaux et le calcul à partir de celle-ci de l'amplitude des signaux.

11. Procédé selon la revendication 10, caractérisé en ce que la courbe d'amplitude des signaux obtenue est comparée à une courbe de référence pour déterminer un temps caractéristique dans la structure de signaux en question, par exemple, son point de fin.

12. Procédé selon l'une quelconque des revendications 1 à 11, caractérisé en ce que lesdites structures de signaux comprennent ce que l'on appelle des potentiels traînants.

13. Dispositif pour traiter des signaux d'ECG dans le but de détecter des structures de signaux à faible amplitude dans du bruit, comprenant des moyens (2, 4) pour enregistrer des signaux d'ECG à partir d'une pluralité de cycles cardiaques, pour la conversion d'analogique en numérique des signaux et pour la détection de complexes QRS, caractérisé en ce qu'une unité de synchronisation est prévue pour synchroniser temporellement les signaux provenant d'au moins deux cycles cardiaques et en ce qu'une unité de calculateur (10) est prévue pour déterminer la concordance entre les signaux dans des intervalles de temps, comprenant au moins deux échantillons et en ce que des moyens de sortie (12) sont connectés à l'unité de calculateur afin de générer une quantité de sortie dont la grandeur désigne l'amplitude des structures de signaux en question.

14. Dispositif selon la revendication 13, caractérisé en ce qu'un circuit de mise en moyenne (6) est prévu pour former au moins deux valeurs de sous-moyenne d'au moins les parties des signaux d'ECG, qui comprennent lesdites structures de signaux, dans au moins deux groupes différents de cycles cardiaques parmi ladite pluralité de cycles cardiaques, ledit circuit de mise en moyenne (6) étant connecté à l'unité de calculateur (10) pour déterminer la concordance entre les valeurs de sous-moyenne.

15. Dispositif selon la revendication 14, caractérisé en ce que l'unité de calculateur (10) est prévue pour former la covariance croisée entre les valeurs de sous-moyenne afin de déterminer la concordance.

16. Dispositif selon la revendication 15, caractérisé en ce que l'unité de calculateur (10) est connectée à une unité de filtre (16) pour filtrer les valeurs de sous-moyenne en conformité avec la grandeur de la covariance croisée et en ce qu'une unité de traitement des signaux (12) est prévue pour soumettre les valeurs filtrées à un traitement non-linéaire dans le but de former une fonction représentant l'amplitude desdites structures de signaux.

17. Dispositif selon la revendication 16, caractérisé en ce qu'une unité (18) est prévue pour estimer le niveau du bruit dans le signal à partir de chaque cycle cardiaque, laquelle unité est connectée à l'unité de traitement des signaux (10) afin de compenser les valeurs de sous-moyenne pour le bruit en liaison avec le traitement des signaux non-linéaire.

18. Dispositif selon l'une quelconque des revendications 15 à 17, caractérisé en ce que l'unité de calculateur (10) est prévue pour calculer la covariance croisée des valeurs de sous-moyenne dans des intervalles successifs se chevauchant.

19. Dispositif selon l'une quelconque des revendications 16 à 18, caractérisé en ce que l'unité de calculateur (10) commande l'unité de filtre (16) afin d'effectuer un filtrage à base de vecteurs propres, commandée par les vecteurs propres déterminés par les calculs de covariance croisée.

20. Dispositif selon l'une quelconque des revendications 16 à 19, caractérisé en ce que l'unité (12) de traitement des signaux est conçue pour élever au carré les valeurs de sous-moyenne filtrées et pour déterminer la puissance du signal et, à partir de celle-ci, l'amplitude du signal.

21. Dispositif selon la revendication 20, caractérisé en ce que l'unité (12) de traitement des signaux comprend ce que l'on appelle un estimateur de Vraisemblance Maximale (32) conçu pour résoudre l'équation de Vraisemblance Maximale

$$\sum_{i=1}^{2m} \lambda_i \left( \frac{1}{\Theta^2 \lambda_i + \sigma_v^2} - \frac{t_i^2}{\left( \Theta^2 \lambda_i + \sigma_v^2 \right)^2} \right) = 0$$

dans laquelle

$\lambda_i$    désigne les valeurs propres de la matrice de covariances croisées,
$\sigma_v^2$   la variance du bruit estimé,
$t_i$    la corrélation entre les valeurs de sous-moyenne et la ième colonne dans la matrice de vecteurs propres,
$\Theta^2$   l'effet du signal, et
m    le nombre d'échantillons dans l'intervalle.

22. Dispositif selon l'une quelconque des revendications 16 à 21, caractérisé en ce qu'une unité de comparateur (14) est connectée à la sortie de l'unité (12) de traitement des signaux afin de comparer la partie terminale de la courbe d'amplitude des signaux à une courbe standard déterminée à l'avance afin de déterminer un temps caractéristique dans la structure de signaux comme la position temporelle dans la courbe de référence qui donne la meilleure concordance avec la courbe d'amplitude des signaux.

## FIG 1

## FIG 2

# FIG 3